(19) Europäisches Patentamt
European Patent Office
Office européen des brevets

(11) Publication number: **0 129 073 B1**

(12) # EUROPEAN PATENT SPECIFICATION

(45) Date of publication of patent specification: **06.03.91**

(51) Int. Cl.⁵: **C12N 15/00**, C07H 21/04, //C12P21/02

(21) Application number: **84105666.6**

(22) Date of filing: **18.05.84**

(54) **Hybrid DNA synthesis of mature growth hormone releasing factor.**

(30) Priority: **25.05.83 US 497776**

(43) Date of publication of application:
**27.12.84 Bulletin 84/52**

(45) Publication of the grant of the patent:
**06.03.91 Bulletin 91/10**

(84) Designated Contracting States:
**AT BE CH DE FR GB IT LI LU NL SE**

(56) References cited:
**EP-A- 0 108 387**

**NATURE, vol. 306, 3rd November 1983, pages 86-88, Macmillan Journals Ltd.; K.E. MAYO et al.: "Expression-cloning and sequence of a cDNA encoding human growth hormone-releasing factor"**

**BIOLOGICAL ABSTRACTS / RRM, abstract no. 26002461, Philadelphia, US; P.J. BARR et al.: "The chemical synthesis and expression of genes for human growth hormone releasing factor somatocrinin" &FEDERATION PROCEEDINGS (USA) 1983, vol. 42, no. 7, abstract 1160**

**PROC. NATL. ACAD. SCI. USA, vol. 80, July 1983, pages 4311-4314; U. GUBLER et al.: "Cloning and sequence analysis of cDNA for the precursor of human growth hormone-releasing factor, somatocrinin"**

(73) Proprietor: **CHIRON CORPORATION
4560 Horton Street
Emeryville California 94608(US)**

(72) Inventor: **Barr, Philip J.
825 Lincoln Way
San Francisco California(US)**
Inventor: **Brake, Anthony J.
2339 Hilgard Avenue
Berkeley California(US)**
Inventor: **Mullenbach, Guy T.
309 63rd Street
Oakland California(US)**

(74) Representative: **Glawe, Delfs, Moll & Partner
Patentanwälte
Rothenbaumchaussee 58 Postfach 2570
W-2000 Hamburg 13(DE)**

**EP 0 129 073 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

#### Field of the Invention

Hybrid DNA technology has made polypeptides accessible, which previously could be obtained only realistically by isolation. Where these polypeptides existed naturally in only small amounts, even isolation became problematic. There was no possibility in these situations to provide the materials in even research amounts, so that they could be studied, no less in amounts which might provide for commercial medical applications. The advent of hybrid DNA technology has changed all this. Now, naturally occurring and synthetic polypeptides can be produced employing the mechanisms of cells, particularly unicellular microorganisms, to produce the polypeptide.

In producing a naturally occurring polypeptide of interest, there are many steps involved. Initially, one must identify a DNA sequence encoding for the polypeptide. After identifying the sequence, which may be quite arduous, one must establish the correctness of the sequence. Where the sequence is obtained as part of a much larger fragment, the fragment must be further manipulated to provide a polynucleotide which can be successfully employed for the subsequent steps of producing a construct which will provide for the desired expression in a host.

An experimental design must be developed based on the codons necessary for expression of the polypeptide and relating the coding sequence to the regulatory and processing signals necessary for expression. The procedural steps will involve various manipulations to provide for the bringing together of DNA fragments from diverse sources which must be correctly oriented and properly juxtaposed to provide for the desired transcriptional regulation and expression of the polypeptide of interest. This frequently involves employing restriction enzymes in the proper order, removal or introduction of restriction sites, joining of the diverse fragments, and isolating the correct construction. Finally, one must establish that the construction is operative in a host to provide the efficient production of the desired polypeptide.

#### Description of the Prior Art

Rivier et al., Nature (1982) 300:276-278; Spiess et al., Biochemistry (1982) 21:6037-6040; and Guillemin et al., Science (1982) 218:585-587 all report the amino acid sequence for growth hormone releasing factor. Brazeau et al., Proc. Natl. Acad. Sci. USA (1982) 79:7909-7913 describe the physiologic activity of the growth hormone releasing factor.

U.S. Patent Nos. 4,336,336 and 4.338.397 describe leader sequences for secretion in prokaryotes. Kurjan and Herskowitz, Cell (1982) 30:933-943 describe a putative α-factor precursor containing four tandem copies of mature α-factor, describing the sequence and postulating a processing mechanism. Kurjan and Herskowitz. Abstracts of Papers Presented at the 1981 Cold Spring Harbor Meeting on the Molecular Biology of Yeasts, p. 242, in an Abstract entitled, "A Putative α-factor Precursor Containing Four Tandem Repeats of Mature α-factor," describe the sequence encoding for the α-factor and spaces between two of such sequences. Blair et al. Abstracts of Papers, ibid., p. 243, in an Abstract entitled, "Synthesis and Processing of Yeast Pheromones: Identification and Characterization of Mutants That Produce Altered α-factors," describe the effect of various mutants on the production of mature α-factor.

### SUMMARY OF THE INVENTION

Methods and compositions are provided for the efficient production of growth hormone release factors which are secreted as matureable or mature polypeptides from a yeast host. The growth hormone releasing factors are prepared in constructs employing the leader and processing signals of yeast secretory signals, where the polypeptide is expressed as a fused protein which is secreted in mature or matureable form.

### DESCRIPTION OF SPECIFIC EMBODIMENTS

In accordance with the subject invention, polypeptides having the physiological activity, of human

growth hormone release factors, are produced. (Unless otherwise indicated when referring to growth hormone release factor polypeptide (s), it is intended to include not only the naturally occurring mammalian factors, but also fragments or analogs thereof exhibiting analogous biological activity.) Procedures are provided for cloning the gene encoding for the growth hormone release factor polypeptides, for preparing constructs for expression of such polypeptides and isolating the polypeptides are precursors to such polypeptides ("pre-polypeptide"). Particularly, the subject polypeptides are prepared as a construct where the subject polypeptides are in reading frame with a leader sequence and processing signals, so that the polypeptides or pre-polypeptides are secreted into the nutrient medium and may be efficiently isolated.

The subject invention involves genes expressing human growth release factor polypeptides, preferably the naturally occurring polypeptides or physiologically active fragments thereof, and novel constructions involving the genes in reading frame with yeast secretory leader processing signals. The constructs will for the most part have the following formula:

5'- LS - (PS-gene) -3'

wherein:

5' and 3' indicate the coding strand direction;

LS is leader sequence for yeast derived from any convenient gene secreted by yeast, such as α-factor, α-factor, acid phosphatase, etc.;

PS is a processing signal which encodes for amino acid sequences recognized by particular peptidases, so that before, during or after secretion, the amino acids are cleaved and removed to provide for the mature polypeptide product, the processing signals may be those associated with any of the polypeptides secreted in yeast, such as the leader sequences indicated above, or may be modified processing signals lacking portions of the processing signal or repeating units; and

gene is the DNA sequence encoding for a growth hormone releasing factor polypeptide and will be described in more detail subsequently.

The secretory leader sequence signal, the processing signal and the gene encoding for the GRF in the same reading frame will define a structural gene encoding for a pre-GRF peptide. The GRF gene, when joined to the processing signal sequence, will usually have an overhang which includes nucleotides of one strand at the 3'-terminus of the processing signal sequence.

Any leader sequence may be employed which provides for secretion. The secretory leader sequence may be a naturally occurring sequence, a fragment of a naturally occurring sequence, a synthetic sequence or combinations thereof. The significant aspect of the secretory leader sequence is its use in combination with the gene, which allows for maturation of the secreted polypeptide. Any processing signals may be employed which permit removal of the secretory leader sequence and in vivo or in vitro removal of the processing signals.

Each of the genes for the growth hormone releasing factor polypeptides or fragment thereof having growth hormone releasing factor physiological activity may be derived from the chromosome, from cDNA, or may be synthesized based on a putative amino acid sequence.

For the purpose of the subject invention, synthetic sequences are employed which are prepared by combining a plurality of single-stranded fragments which have been analyzed and prepared to be devoid of significant stretches of repeating sequences, that is, the absence of repeating sequences of more than 9 bases, preferably of not more than 7 bases. In addition, the sequences are designed, so that restriction endonuclease recognition sites of restriction enzymes which may be subsequently used in the preparation of the expression vector will be absent, preferably there will be not more than one restriction endonuclease recognition site in the coding sequence, more preferably there being no restriction endonuclease recognition site of a known restriction endonuclease. Furthermore, since the expression product is to be prepared in yeast, it is desirable that the codons be those which are preferred by yeast. By being preferred codons is intended the frequency with which a particular codon appears for encoding the yeast glycolytic enzymes.

The genes can be prepared by bringing together a plurality of single-stranded sequences varying from about 8 to 40, more usually from about 10 to 35 bases, where overlap occurs of at least 5 bases, more usually at least about 8 bases. Desirably, the terminal single strands provide for overhangs or cohesive ends which can be joined to the secretory and processing signals at the 5'-end of the coding strand and 3'-transcriptional regulatory signals, e.g. polyadenylation signal, transcriptional terminator, etc., at the 3'-end of the coding strand. Stop codons for translational termination may also be included in the synthetic sequence or may be present in a different sequence to be subsequently joined to the synthetic gene.

Synthetic genes of particular interest have the following DNA sequences:

## EXHIBIT 1

GRF-40:

```
     AlaTyrAlaAspAlaIlePheThrAsnSerTyrArgLysValLeuGlyGlnLeuSerAla
    AGCTTACGCTGACGCTATCTTCACCAACTCCTACAGAAAGGTTTTGGGTCAATTGTCCGCT
        ATGCGACTGCGATAGAAGTGGTTGAGGATGTCTTTCCAAAACCCAGTTAACAGGCGA

     ArgLysLeuLeuGlnAspIleMetSerArgGlnGlnGlyGluSerAsnGlnGluArgGly
 61  AGAAAGTTGTTGCAAGACATCATGTCCAGACAACAAGGTGAATCCAACCAAGAAAGAGGT
     TCTTTCAACAACGTTCTGTAGTACAGGTCTGTTGTTCCACTTAGGTTGGTTCTTTCTCCA

     AlaOP OC
121  GCTTGATAAG
     CGAACTATTCAGCT
```

GRF-44:

```
     AlaTyrAlaAspAlaIlePheThrAsnSerTyrArgLysValLeuGlyGlnLeuSerAla
    AGCTTACGCTGACGCTATCTTCACCAACTCCTACAGAAAGGTTTTGGGTCAATTGTCCGCT
        ATGCGACTGCGATAGAAGTGGTTGAGGATGTCTTTCCAAAACCCAGTTAACAGGCGA

     ArgLysLeuLeuGlnAspIleMetSerArgGlnGlnGlyGluSerAsnGlnGluArgGly
 61  AGAAAGTTGTTGCAAGACATCATGTCCAGACAACAAGGTGAATCCAACCAAGAAAGAGGT
     TCTTTCAACAACGTTCTGTAGTACAGGTCTGTTGTTCCACTTAGGTTGGTTCTTTCTCCA

     AlaArgAlaArgLeuOC AM IleOP IleOP OC
121  GCTAGAGCTAGATTGTAATAGATCTGAATTTGATAAG
     CGATCTCGATCTAACATTATCTAGACTTAAACTATTCAGCT
```

wherein,

OP intends opal codon;
OC intends ochre codon; and
AM intends amber codon.

In the above constructions, the 5'- end overhang provides for binding to a Hin dIII recognition site overhang while the 3'-end overhang provides for binding to a SalI recognition site overhang.

The synthetic gene may be inserted into a construct to produce an expression vector or gene containing sequence having the following formula:

- P $(ti)_a$ - LS - PS - gene - tt - T - $|RepS|_n$ -

wherein:

each of the functional sequences symbolized by the symbols may be separated by extended sequences where such sequences do not interfere with the function of the symbolized sequence;

P intends the promoter or RNA polymerase binding site;

ti intends transcriptional initiation regulation site or sequences which includes various DNA sequences associated with the initiation of transcription and processing of the messenger RNA, such as recognition sites for repressors (operators), enhancers, activators, derepressors, cap site, or the like, which may be subject to extrinsic regulation by temperature, small organic molecules, etc.;

LS is a yeast recognized leader sequence including the secretory leader signal, particularly α-factor and α-factor;

PS is the processing signal(s) which includes at least two basic amino acids, which may be the same or different, and are lysine or arginine, and may include additional dipeptide sequences, such as glutamine-alanine, glutamic acid-proline, aspartic acid-alanine, or as described further below;

gene intends a DNA sequence which encodes for a polypeptide of at least about 20, more usually at least about 29, and generally either 40 or 44 amino acids and not more than about 45 amino acids capable of having one or more of the physiological functions of growth hormone releasing factor, more particularly N-terminal fragments of less than 45 amino acids;

tt intends transcriptional termination recognition site or sequences, which includes stop codons, polyadenylation signals, or the like;

T intends the transcriptional terminator;

RepS intends the replication system which includes one or more DNA sequences encoding for replication by at least a yeast host and additionally one or more other hosts, particularly prokaryotic hosts, more particularly E. coli;

a is 0 or 1; and

n is 1 to 3, more usually 1 to 2.

Various processing signals may be employed having at least one dipeptide unit of basic amino acids and usually not more than about three such units, where the basic amino acid dipeptide units may be joined together or separated by one or more, usually two or more amino acids, where the intervening amino acids may be one to three dipeptide units. The basic amino acids are lysine and arginine. Intervening dipeptide units may be:

X - Y

wherein:

X is an acidic amino acid or amide thereof, i.e. asp, glu, asn, and gln; and

Y is an acidic amino acid or amide thereof or ala or pro.

In addition to the sequences described above, other sequences may encode for other structural genes, particularly markers, which are recognized by the host(s) for the replication system(s), which allows for detection of those host cells containing the gene of interest. These structural genes referred to as markers may provide for prototrophy in an auxotrophic host; biocidal resistance, such as from antibiotics, heavy metals, or toxins; immunity, or the like. One or more markers may be present, usually not more than 3, more usually not more than 2.

In preparing the constructs of the subject invention for expression of human growth hormone releasing factor or analogs thereof, the gene may be synthetically prepared by employing single strands as described above, which are combined under ligating conditions, where the sequences require that the single strands align and anneal to provide for the desired coding sequence. In addition, as indicated, the single strands can provide for specific cohesive ends which allow for proper alignment and reading frame with the secretory leader sequence and processing signals and joining to the appropriate transcriptional and translational termination signals.

By appropriate choice of the upstream overhang (5'- or 3'-) one can provide for linking of the subject gene to leader and processing signals in proper reading frame. Particularly, with α-factor, the upstream 5'-overhang can have a 5'- terminal base sequence which provides for a portion of the Hin dIII recognition sequence. The α-factor processing signals have a convenient Hin dIII site, so that upon linking of the α-factor secretion leader and processing signals with the synthetic gene, the processing signals are recreated and the gene is in reading phase with the secretory leader sequence.

In addition, the downstream terminus can be chosen to link with an appropriate restriction site overhang, conveniently in the present case Sal I, so that a terminator is present, as well as a polyadenylation coding sequence. Where a leader sequence is employed, it will usually be convenient to also have the promoter associated with the leader sequence. However, if desired, additional promoters can be joined to the leader sequence promoter or the leader sequence promoter may be substituted by one or more other promoters. A wide variety of promoters recognized by yeast have been reported as isolated, particularly the glycolytic enzyme promoters. See, for example, Hitzeman et al., J. Biol. Chem. (1980) 255 :12073.

The expression vector may have one or more replication systems, particularly where one replication system is for bacteria, to allow for cloning during the construction, while the other replication system is for the host in which the gene is to be expressed. with the α-factor or a -factor secretion leader sequences and processing signals, yeast would be the normal host, although other hosts which recognize the signals could be employed.

After each manipulation the resulting construct may be cloned in a prokaryotic host and the desired DNA fragment isolated by restriction cleavage and used for further manipulation.

The expression construct or plasmid may be introduced into an appropriate host, such as S . cerevisiae or other yeast strain. For cloning, the plasmid may be cloned in E . coli. Various techniques are known for introducing DNA into a host, such as calcium chloride treatment of cells, liposomes, cosmids, or the like. Once the DNA has been introduced into the host, the host is grown under conditions which select for those cells containing the desired plasmid. The desired oligopeptide will be expressed and processed, so that a processed oligopeptide having the desired amino acid sequence may be obtained in the nutrient medium.

Various mutant hosts may be employed having desired mutations or hosts may be modified with episomal elements or by integration to impart desirable characteristics. For example, desirable hosts may overproduce particular enzymes, e.g. membrane enzymes involved in processing, such as dipeptides, may provide controllable regulation of particular promoters, e.g. temperature-sensitive repression, may have desirable growth characteristics, membrane characteristics, or the like.

The following examples are offered by way of illustration and not by way of limitation:

EXPERIMENTAL

Nucleotide sequences for human growth hormone releasing factors -40 and -44 comprising preferred yeast codons and eliminating extended repeat sequences were devised based on the amino acid sequence described in Rivier et al ., Nature (1982) 300 :276-278 and Guillemin et al ., Science (1982) 218:585-587, respectively. Single-stranded sequences were prepared, which could be combined in a single medium, annealed and ligated to provide the complete gene with appropriate termini. The following sequences with the coding strands shown 5' to 3' were prepared in accordance with conventional synthetic techniques.

| Sequences (5'-3') | |
|---|---|
| H-1-13mer | AGCTTACGCTGAC |
| H-2-25mer | GCTATCTTCACCAACTCCTACAGAA |
| H-3-25mer | AGGTTTTGGGTCAATTGTCCGCTAG |
| H-4-25mer | AAAGTTGTTGCAAGACATCATGTCC |
| H-5-25mer | AGACAACAAGGTGAATCCAACCAAG |
| H-6-34mer | AAAGAGGTGCTAGAGCTAGATTGTAATAGATCTG |
| H-7-21mer | GGTGAAGATAGCGTCAGCGTA |
| H-8-25mer | GACCCAAAACCTTTCTGTAGGAGTT |
| H-9-25mer | TGCAACAACTTTCTAGCGGACAATT |
| H-10-25mer | ACCTTGTTGTCTGGACATGATGTCT |
| H-11-24mer | AGCACCTCTTTCTTGGTTGGATTC |
| H-12-27mer | AATTCAGATCTATTACAATCTAGCTCT |
| H-13-18mer | AAAGAGGTGCTTGATAAG |
| Linker-1 | AATTTGATAAG |
| Linker-2 | TCGACTTATCA |

The various oligonucleotides joined in accordance with the ligation strategy are shown in Table 1.

TABLE 1

**Ligation Strategy**

**GRF(1-44):**

H-1(13) H-2(25) H-3(25) H-4(25) H-5(25) H-6(34) Linker 1

H-7(21) H-8(25) H-9(25) H-10(25) H-11(24) H-12(27) Linker 2

**GRF(1-40):**

H-1(13) H-2(25) H-3(25) H-4(25) H-5(25) H-13(18)

H-7(21) H-8(25) H-9(25) H-10(25) H-11(24) Linker 2

The completed genes with appropriate flanking nucleotides and the encoded amino acids are as indicated in Exhibit 1.

The ssDNA segments were joined as follows: 1 nmole of each segment was 5'-phosphorylated with T4 polynucleotide kinase. Two hundred pmoles of all the phosphorylated segments were then annealed in a single step as an 30µl pool by cooling from 95°C to 25°C over approximately 1.5 hours. Ligation was achieved in a reaction volume of 30µl containing 3mM ATP, 10mM DTT, 100mM tris-HCl, pH 7.8, 10mM $MgCl_2$, 1µg ml spermidine and T4 DNA ligase. The double-stranded fragments resulting from the above reaction mixture were digested with Hin dIII and SalI and then purified on a 7% native polyacrylamide electrophoresis gel (PAGE).

The joining of the synthetic nucleotide sequences encoding GRF, described above, to the sequences

encoding yeast α-factor, was accomplished as follows. First, a yeast genomic library was obtained by cloning Sau3A restriction fragments of yeast DNA into the BamHI site of the plasmid YEp24. Plasmid pAB101 which contains the yeast α-factor gene as a partial Sau3A fragment, was obtained by screening the library with an enzymatically $^{32}P$ radiolabeled synthetic oligonucleotide probe homologous to the published α-factor coding region (Kurjan and Herskowitz, Abstracts 1981 Cold Spring Harbor Meeting on the Molecular Biology of Yeast, p. 242). Digestion of pAB101 with EcoRI yields a 1.8kb fragment containing the α-factor gene. This EcoRI fragment was cloned into the EcoRI site of pBR 322, from which the HindIII and SalI sites were deleted, yielding pAB112. Treatment of pAB112 with HindIII causes the deletion of three 63bp fragments, and yields pAB113. The purified synthetic fragments encoding GRF, the preparation of which is described above, were ligated to 100 ng of pAB113, which had been previously digested to completion with endonucleases HindIII and SalI.

The resulting mixtures were used to transform E. coli HB101 cells and plasmids PAB113/GRF40.1 or 40.1A1 and pAB113/GRF44/18 or 20 were obtained for GRF(1-40) and GRF(1-44), respectively.

The two plasmids (5µg each) were digested to completion with Eco RI and the resulting fragments were ligated to an excess of Eco RI-Bam HI adaptors and digested with Bam HI. The resulting Bam HI fragments were isolated by a preparative gel electrophoresis and approximately 100ng of each fragment is ligated to 100ng of pc1/1, which has been previously digested to completion with Bam HI and treated with alkaline phosphatase.

Plasmid pc1/1 is a derivative of pJDB219, Beggs, Nature (1978) 275 :104, in which the region corresponding to bacterial plasmids pMB9 in pJDB219 has been replaced by pBR322.

Each ligation mixture is used to transform E . coli HB101 cells. Transformants are selected by ampicillin resistance and the plasmids analyzed by restriction endonuclease digestion. DNA from one selected clone from each structural gene, pC1/1 GRF44/18-6 or 20-1 and pC1/1 GRF40.1/6 or 40.1A-1 7, are prepared and used to transform yeast AB103 cells. Transformants are selected by the $Leu^+$ phenotype.

Cultures (100ml for GRF40 and 50ml for GRF44) of yeast strain AB103 (α, pep 4 -3 , leu 2 -3 , leu 2 - 112 , ura 3 -52 , his 4 -580 ) are transformed with plasmids and grown at 33°C in -leu (medium lacking leucine) medium to saturation (optical density at 650nm of 5) and are left shaking at 30°C for an additional 12 hour period. Cell supernatants are collected from each culture by centrifugation and the polypeptide concentrated by absorption on an ion exchange resin (Bio-rex-70 available from Bio-Rad Laboratories, Inc., Richmond, California). After elution with 10mM HCl in 80% ethanol, the peptide fractions are then evaporated to dryness, taken up in 100µl $H_2O$ (GRF-44) or 50µl $H_2O$ (GRF-40) and then assayed for GRF concentration. The plasmids pC1/1 GRF40.1/6 and pC1/1 GRF44/18-6 gave yields of 2-5mg/ml of polypeptides having the GRF sequence. With GRF40.1/6 90% has (glu-ala) at the N-terminus as determined by the amino acid sequence; the remaining 10% of the material had the native sequence. The protein assay is the Coomassie Blue assay available from Bio-Rad Laboratories, Inc.

In accordance with the subject invention, novel constructs are provided including sequences encoding for growth hormone releasing factor, particularly joined in reading frame with secretory and processing leader signals, so as to provide for secretion of the polypeptide product into the nutrient medium. By virtue of providing for secretion, greatly enhanced yields can be obtained based on self-population and subsequent preparative operations and purification are simplified.

Although the foregoing invention has been described in some detail by way of illustration and example for purposes of clarity of understanding, it will be obvious that certain changes and modifications may be practiced within the scope of the appended claims.

## Claims

**1.** A method for production of a polypeptide having human growth hormone releasing factor (GRF) physiological activity, comprising:

a. growing yeast host cells containing a functional recombinant DNA construct having a nucleotide sequence (NS1) encoding the GRF polypeptide, downstream of and joined in proper reading frame with a nucleotide sequence (NS2) encoding a peptide containing yeast secretory leader and processing signals derived from alpha factor, and also having a transcriptional regulatory region which regulates the transcription of NS1 and NS2; and

b. isolating the secreted polypeptide.

**2.** A method as in claim 1 wherein the sequence encoding the GRF peptide is a synthetic sequence

having codons preferentially utilized by the yeast host.

3. A method as in claim 2 wherein the nucletoide sequence encoding the GRF peptide is the following, with the amino acid sequence indicated in conjunction with the codons of the nucleotide sequence:

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. A method as in claim 2 wherein the nucleotide sequence encoding the GRF peptide is the following, with the amino acid sequence indicating in conjunction with the codons of the nucleotide sequence:

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. A method according to claim 1 wherein the vector is a derivative of the 2μm plasmid.

6. A method according to claim 1 wherein said GRF is GRF-40.

7. A method according to claim 1 wherein said GRF is GRF-44.

8. A functional DNA construct for use in a method according to claim 1, comprised of a nucleotide sequence (NS1) encoding the GRF polypeptide, downstream of and joined in proper reading frame with a nucleotide sequence (NS2) encoding a peptide containing yeast secretory leader and processing signals derived from alpha factor, and also having a transcriptional regulatory region which regulates the transcription of NS1 and NS2.

9. A DNA construct according to claim 8 wherein said sequence encoding GRF encodes for GRF-40.

10. A DNA construct according to claim 8 wherein said coding sequence encoding GRF encodes for GRF-44.

11. A DNA construct according to claim 8 wherein said DNA construct is joined to a replication system

utilized by yeast.

12. A DNA construct according to claim 9 wherein the sequence encoding GRF has the following sequence with the amino acid sequence indicated in conjunction with the codons of the nucleotide sequence:

```
  AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

  ArgLysLeuLeuGlnAspIleMetSerArgGlnGlnGlyGluSerAsnGlnGluArgGly
  AGAAAGTTGTTGCAAGACATCATGTCCAGACAACAAGGTGAATCCAACCAAGAAAGAGGT
  TCTTTCAACAACGTTCTGTAGTACAGGTCTGTTGTTCCACTTAGGTTGGTTCTTTCTCCA

  AlaOP OC
  GCTTGATAAG
  CGAACTATTCAGCT
```

13. A DNA construct according to claim 10 wherein the sequence encoding GRF has the following sequence with the amino acid sequence indicated in conjunction with the codons of the nucleotide sequence:

```
  AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

  ArgLysLeuLeuGlnAspIleMetSerArgGlnGlnGlyGluSerAsnGlnGluArgGly
  AGAAAGTTGTTGCAAGACATCATGTCCAGACAACAAGGTGAATCCAACCAAGAAAGAGGT
  TCTTTCAACAACGTTCTGTAGTACAGGTCTGTTGTTCCACTTAGGTTGGTTCTTTCTCCA

  AlaArgAlaArgLeuOC AM IleOP IleOP OC
  GCTAGAGCTAGATTGTAATAGATCTGAATTTGATAAG
  CGATCTCGATCTAACATTATCTAGACTTAAACTATTCAGCT
```

Claims for Contracting State: AT

1. A method for production of a polypeptide having human growth hormone releasing factor (GRF) physiological activity, comprising:
   a. growing yeast host cells containing a functional recombinant DNA construct having a nucleotide sequence (NS1) encoding the GRF polypeptide, downstream of and joined in proper reading frame with a nucleotide sequence (NS2) encoding a peptide containing yeast secretory leader and processing signals derived from alpha factor, and also having a transcriptional regulatory region which regulates the transcription of NS1 and NS2; and
   b. isolating the secreted polypeptide.

2. A method as in claim 1 wherein the sequence encoding the GRF peptide is a synthetic sequence having codons preferentially utilized by the yeast host.

3. A method as in claim 2 wherein the nucletoide sequence encoding the GRF peptide is the following, with the amino acid sequence indicated in conjunction with the codons of the nucleotide sequence:

```
  AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. A method as in claim 2 wherein the nucleotide sequence encoding the GRF peptide is the following, with the amino acid sequence indicating, in conjunction with the codons of the nucleotide sequence:

```
  AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. A method according to claim 1 wherein the vector is a derivative of the 2µm plasmid.

6. A method according to claim 1 wherein said GRF is GRF-40.

7. A method according to claim 1 wherein said GRF is GRF-44.

**Revendications**

1. Procédé de production d'un polypeptide ayant une activité physiologique de facteur libérant de l'hormone de croissance humaine (GRF), comprenant :
   a) la culture de cellules de levure hôtes contenant un produit d'assemblage d'ADN recombinant fonctionnel ayant une séquence de nucléotides ($SN_1$) codant pour le polypeptide GRF, en aval et réunie en phase de lecture correcte avec une séquence de nucléotides ($SN_2$) codant pour un peptide contenant une séquence leader de sécrétion de la levure et des signaux de maturation dérivés du facteur α et ayant également une région de régulation de transcription qui régule la transcription de $NS_1$ et $NS_2$ : et
   b) l'isolement du polypeptide sécrété.

2. Procédé selon la revendication 1, dans lequel la séquence codant pour le peptide GRF est une séquence de synthèse ayant des codons utilisés préférentiellement par la levure hôte.

3. Procédé selon la revendication 2, dans lequel la séquence de nucléotides codant pour le peptide GRF

est la suivante, la séquence d'amino-acides étant indiquée en correspondance avec les codons de la séquence de nucléotides :

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. Procédé selon la revendication 2, dans lequel la séquence de nucléotides codant pour le peptide GRF est la suivante, la séquence d'amino-acides étant indiquée en correspondance avec les codons de la séquence de nucléotides :

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. Procédé selon la revendication 1, dans lequel le vecteur est un dérivé du plasmide 2$\mu$m.

6. Procédé selon la revendication 1, dans lequel ledit GRF est le GRF-40.

7. Procédé selon la revendication 1, dans lequel ledit GRF est le GRF-44.

8. Produit d'assemblage d'ADN fonctionnel pour l'utilisation dans un procédé selon la revendication comprenant une séquence de nucléotides ($SN_1$) codant pour le polypeptide GRF, en aval et réunie en phase de lecture correcte avec une séquence de nucléotides ($SN_2$) codant pour un peptide contenant une séquence leader de sécrétion de la levure et des signaux de maturation dérivés du facteur $\alpha$, et ayant également une région de régulation de transcription qui régule la transcription de $NS_1$ et $NS_2$.

9. Produit d'assemblage d'ADN selon la revendication 8, dans lequel ladite séquence codant pour GRF code pour GRF-40.

10. Produit d'assemblage d'ADN selon la revendication 8, dans lequel ladite séquence codant pour GRF code pour GRF-44.

11. Produit d'assemblage d'ADN selon la revendication 8, dans lequel ledit produit d'assemblage est réuni

à un système de réplication utilisé par la levure.

**12.** Produit d'assemblage d'ADN selon la revendication 9, dans lequel la séquence codant pour GRF a la séquence suivante, la séquence d'amino-acides étant indiquée en correspondance avec les codons de la séquence de nucléotides :

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

**13.** Produit d'assemblage d'ADN selon la revendication 10, dans lequel la séquence codant pour GRF a la séquence suivante, la séquence d'amino-acides étant indiquée en correspondance avec les codons de la séquence de nucléotides :

```
   AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

**1.** Procédé de production d'un polypeptide ayant une activité physiologique de facteur libérant de l'hormone de croissance humaine (GRF), comprenant :

a) la culture de cellules de levure hôtes contenant un produit d'assemblage d'ADN recombinant fonctionnel ayant une séquence de nucléotides ($SN_1$) codant pour le polypeptide GRF, en aval et réunie en phase de lecture correcte avec une séquence de nucléotides ($SN_2$) codant pour un peptide contenant une séquence leader de sécrétion de la levure et des signaux de maturation dérivés du facteur α, et ayant également une région de régulation de transcription qui régule la transcription de $NS_1$ et $NS_2$ ; et

b) l'isolement du polypeptide sécrété.

**2.** Procédé selon la revendication 1, dans lequel la séquence codant pour le peptide GRF est une séquence de synthèse ayant des codons utilisés préférentiellement par la levure hôte.

**3.** Procédé selon la revendication 2, dans lequel la séquence de nucléotides codant pour le peptide GRF est la suivante, la séquence d'amino-acides étant indiquée en correspondance avec les codons de la séquence de nucléotides :

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. 4. Procédé selon la revendication 2, dans lequel la séquence de nucléotides codant pour le peptide GRF est la suivante, la séquence d'amino-acides étant indiquée en correspondance avec les codons de la séquence de nucléotides :

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. Procédé selon la revendication 1, dans lequel le vecteur est un dérivé du plasmide 2µm.

6. Procédé selon la revendication 1, dans lequel ledit GRF est le GRF-40.

7. Procédé selon la revendication 1, dans lequel ledit GRF est le GRF-44.

## Ansprüche

1. Verfahren zur Herstellung eines Polypeptids mit einer physiologischen Aktivität des Wachstumshormon-Freisetzungs-Faktors (GRF), mit den Merkmalen:
   a) Züchten von Hefewirtszellen, die ein funktionelles rekombiniertes DNA-Konstrukt enthalten, das eine das GRF-Polypeptid kodierende Nukleotidsequenz (NS1) enthält und stromabwärts von sowie in einem geeigneten Leseraster zu einer ein Peptid mit von dem α-Faktor abgeleiteten Hefe-Sekretions-Leader- und -Processing-Signalen kodierenden Nukleotidsequenz (NS2) angeordnet bzw. verknüpft ist, und das weiterhin eine die Transkription von NS1 und NS2 regulierende transkriptionelle Regulationsregion enthält; und
   b) Isolieren des sekretierten Polypeptids.

2. Verfahren nach Anspruch 1, in dem die das GRF-Peptid kodierende Sequenz eine synthetische Sequenz ist, die vorzugsweise von dem Hefewirt verwendete Kodons aufweist.

3. Verfahren nach Anspruch 2, in dem die das GRF-Peptid kodierende Nukleotidsequenz die folgende ist,

wobei zusammen mit den Kodons der Nukleotidsequenz die Aminosäuresequenz gezeigt ist:

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. verfahren nach Anspruch 2, in dem die das GRF-Peptid kodierende Nukleotidsequenz die folgende ist, wobei zusammen mit den Kodons der Nukleotidsequenz die Aminosäuresequenz gezeigt ist:

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. Verfahren nach Anspruch 1, in dem der Vektor ein Derivat des 2 µm-Plasmids ist.

6. Verfahren nach Anspruch 1, in dem GRF GRF-40 ist.

7. Verfahren nach Anspruch 1, in dem GRF GRF-44 ist.

8. Funktionelles DNA-Konstrukt zur Verwendung in einem Verfahren nach Anspruch 1, das eine das GRF-Polypeptid kodierende Nukleotidsequenz (NS1) enthält und stromabwärts von sowie in einem geeigneten Leseraster zu einer ein Peptid mit von dem α-Faktor abgeleiteten Hefe-Sekretions-Leader-und -Processing-Signalen kodierenden Nucleotidsequenz (NS2) angeordnet bzw. verknüpft ist, und das weiterhin eine die Transkription von NS1 und NS2 regulierende transkriptionelle Regulationsregion enthält.

9. DNA-Konstrukt nach Anspruch 8, in dem die GRF kodierende Sequenz GRF-40 kodiert.

10. DNA-Konstrukt nach Anspruch 8, in dem die GRF kodierende Kodierungssequenz GRF-44 kodiert.

11. DNA-Konstrukt nach Anspruch 8, verbunden mit einem von Hefe verwendeten Replikationssystem.

12. DNA-Konstrukt nach Anspruch 9, in dem die das GRF-Peptid kodierende Nukleotidsequenz die folgende ist, wobei zusammen mit den Kodons der Nukleotidsequenz die Aminosäuresequenz gezeigt ist:

AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT

13. DNA-Konstrukt nach Anspruch 10, in dem die das GRF-Peptid kodierende Nukleotidsequenz die folgende ist, wobei zusammen mit den Kodons der Nukleotidsequenz die Aminosäuresequenz gezeigt ist:

AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT

Patentansprüche für den Vertragsstaat: AT

1. Verfahren zur Herstellung eines Polypeptids mit einer physiologischen Aktivität des Wachstumshormon-Freisetzungs-Faktors (GRF), mit den Merkmalen:
   a) Züchten von Hefewirtszellen, die ein funktionelles rekombiniertes DNA-Konstrukt enthalten, das eine das GRF-Polypeptid kodierende Nukleotidsequenz (NS1) enthält und stromabwärts von sowie in einem geeigneten Leseraster zu einer ein Peptid mit von dem α-Faktor abgeleiteten Hefe-Sekretions-Leader- und -Processing-Signalen kodierenden Nukleotidsequenz (NS2) angeordnet bzw. verknüpft ist, und das weiterhin eine die Transkription von NS1 und NS2 regulierende transkriptionelle Regulationsregion enthält: und
   b) Isolieren des sekretierten Polypeptids.

2. Verfahren nach Anspruch 1, in dem die das GRF-Peptid kodierende Sequenz eine synthetische Sequenz ist, die vorzugsweise von dem Hefewirt verwendete Kodons aufweist.

3. Verfahren nach Anspruch 2, in dem die das GRF-Peptid kodierende Nukleotidsequenz die folgende ist, wobei zusammen mit den Kodons der Nukleotidsequenz die Aminosäuresequenz gezeigt ist:

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaOP OC
GCTTGATAAG
CGAACTATTCAGCT
```

4. Verfahren nach Anspruch 2, in dem die das GRF-Peptid kodierende Nukleotidsequenz die folgende ist, wobei zusammen mit den Kodons der Nukleotidsequenz die Aminosäuresequenz gezeigt ist:

```
 AlaTyrAlaAspAlaIlePheThrAsnSerTyrArgLysValLeuGlyGlnLeuSerAla
AGCTTACGCTGACGCTATCTTCACCAACTCCTACAGAAAGGTTTTGGGTCAATTGTCCGCT
    ATGCGACTGCGATAGAAGTGGTTGAGGATGTCTTTCCAAAACCCAGTTAACAGGCGA

ArgLysLeuLeuGlnAspIleMetSerArgGlnGlnGlyGluSerAsnGlnGluArgGly
AGAAAGTTGTTGCAAGACATCATGTCCAGACAACAAGGTGAATCCAACCAAGAAAGAGGT
TCTTTCAACAACGTTCTGTAGTACAGGTCTGTTGTTCCACTTAGGTTGGTTCTTTCTCCA

AlaArgAlaArgLeuOC AM IleOP IleOP OC
GCTAGAGCTAGATTGTAATAGATCTGAATTTGATAAG
CGATCTCGATCTAACATTATCTAGACTTAAACTATTCAGCT
```

5. Verfahren nach Anspruch 1, in dem der Vektor ein Derivat des 2 µm-Plasmids ist.

6. Verfahren nach Anspruch 1, in dem GRF GRF-40 ist.

7. Verfahren nach Anspruch 1, in dem GRF GRF-44 ist.